# EUROPÄISCHE PATENTSCHRIFT

(11) **EP 1 267 932 B1**
(45) Veröffentlichungstag und Bekanntmachung des Hinweises auf die Patenterteilung: **18.02.2004**
(21) Anmeldenummer: 01919354.9
(22) Anmeldetag: 28.02.2001
(51) Int. Cl.: A61K 45/06, A61P 31/10

(54) **ANTIINFEKTIVE WIRKSTOFFKOMBINATIONEN UND IHRE VERWENDUNG ZUR TOPISCHEN BEHANDLUNG VON PILZERKRANKUNGEN DER FUSS- UND FINGERNÄGEL**
ANTI-INFECTIVE ACTIVE SUBSTANCE COMBINATIONS AND THE USE THEREOF FOR THE TOPICAL TREATMENT OF FUNGUS DISEASES OF TOE AND FINGER NAILS
COMBINAISONS ANTI-INFECTIEUSES DE PRINCIPES ACTIFS ET LEUR UTILISATION DANS LE TRAITEMENT TOPIQUE DE MYCOSES DES ONGLES DES ORTEILS ET DES DOIGTS

(30) Priorität: 09.03.2000 DE 10011081
(43) Veröffentlichungstag der Anmeldung: 02.01.2003
(73) Patentinhaber: Aventis Pharma Deutschland GmbH, 65929 Frankfurt am Main (DE)
(72) Erfinder: BOHN, Manfred, 65719 Hofheim (DE); KRAEMER, Karl, Theodor, 63225 Langen (DE)
(86) Internationale Anmeldenummer: PCT/EP2001/002236
(87) Internationale Veröffentlichungsnummer: WO 2001/066145

(56) Entgegenhaltungen:
- EP-A- 0 515 312
- WO-A-87/02580
- WO-A-99/39680
- WO-A-99/49835

## Beschreibung

Pilzerkrankungen der Fuß- und Fingernägel (Onychomykosen) sind weltweit verbreitet. Gerade in den hochentwickelten Industrienationen nimmt dieses chronische, nicht zur Selbstheilung neigende Krankheitsbild einen immer größeren Stellenwert ein. Onychomykosen stellen mit einem Anteil von bis zu 40% die häufigste Nagelerkrankung dar. Die Prävalenz der Onychomykosen wird im Stand der Technik mit 2,8 % bis 8,4 % angegeben. Nagelmykosen machen heute etwa 30 % aller Dermatomykosen aus. Epidemiologische Studien zeigen, dass 20 % bis 30% der Patienten mit Tinea pedis auch eine Onychomykose aufweisen.

Viele Patienten fühlen sich, insbesondere wenn die Onychomykose deutlich sichtbar an den Fingernägeln lokalisiert ist, in ihren Sozialkontakten eingeschränkt. Darüber hinaus kommt es infolge des Krankheitsgeschehens zu einer möglichen Einschränkung der Taktilität, der Motilität und der manuellen Fähigkeiten. Behandlungsbedarf ergibt sich auch aus der Tatsache, dass Onychomykosen als Infektionsquelle zur Ausbreitung der Krankheit vom Nagel auf die freie Haut beitragen. Ferner stellen Sie eine Infektionsgefahr für eine ständig zunehmende Population dar.

Seit dem Beginn der neunziger Jahre ist eine Vielzahl von neuen Behandlungsmethoden zur Therapie der Onychomykose entwickelt worden. Dies sind einerseits neue systemisch wirksamen Antimykotika; beispielsweise Itraconazol, siehe US 4,267,179; (E)-N-(6,6-Dimethyl-2-hepten-4-yn-yl)-N-methyl-1-naphthalen methanamin, das auch als Terbinafin bezeichnet wird, und α-(2,4-Difluorphenyl)-α-(1H-1,2,4-triazol-1-ylmethyl)-1H-1,2,4-triazol-1-ethanol, das auch als Fluconazol bezeichnet wird, aber auch topisch anzuwendende Lackzubereitungen, die die Behandlung von Onychomykosen erfolgversprechender gestalten.

Die systemische Therapie kann erfahrungsgemäß bisweilen zu gravierenden, unerwünschten, unter Umständen lebensbedrohenden, Arzneimittelnebenwirkungen führen, da der Wirkstoff über den Blutkreislauf zu dem Infektionsherd gelangen muß.

Neben- und Wechselwirkungen mit anderen Medikamenten sind gerade bei vielen älteren, multimorbiden Patienten vorprogrammiert. Systemische Antimykotika verfügen darüber hinaus über weitere unerwünschte Begteiteffekte wie Lücken im Spektrum der behandelbaren Erreger oder eine teilweise unsichere Resorption.

In jüngster Zeit wurden verschiedene Studien mit Antimykotika-haltigen Lackzubereitungen in Kombination mit einer systemischen Itraconazol- bzw. Terbinafin-Therapie bei Patienten mit ausgeprägter Onychomykose durchgeführt. Die Ergebnisse zeigen, dass bei der Therapie von schweren Onychomykosen die Kombination einer topischen Lackzubereitung mit einer systemischen Gabe von Itraconazol oder Terbinafin der Monotherapie mit Itraconazol und Terbinafin deutlich überlegen ist. Die bisherigen Resultate deuten darauf hin, dass die Misserfolgsrate bei systemischer Therapie von Onychomykosen durch die Kombinationstherapie mit einer topisch wirksamen Antimykotika-haltigen Lackzubereitung und einem systemisch wirksamen Antimykotikum erheblich reduziert werden kann.

Nachteil der kombinierten Behandlung mit einem topischen und einem systemischen Antimykotikum in der Therapie von Onychomykosen bleibt die systemische Belastung mit allen damit verbundenen negativen Effekten, die für den Patienten auch bei dieser Behandlungsstrategie bestehen. Ein weiterer gewichtiger Nachteil ist die geringe Menge an systemischen Antimykotikum, die den Fuß- oder Fingernagel erreicht. Ferner ist es bisher nur mit sehr aufwendigen und drastischen Methoden gelungen systemische Antimykotika wie Itraconazol in therapeutisch wirksamen Konzentrationen topisch auf den Fuß- oder Fingernagel zu applizieren (siehe WO 96/19186).

Aufgabe der vorliegenden Erfindung ist es eine Formulierung zur Verfügung zu stellen, die die bekannten Nachteile bei der beschriebenen topisch/systemischen Kombinationstherapie von Onychomykosen nicht aufweist.

Entgegen dem bestehenden technischen Vorurteil, dass systemische Antimykotika, wie Itraconazol, bei topischer Applikation nur nach Auflösung der Schwefelbrücken des Nagelkeratins und durch Zugabe von Harnstoff in ausreichender Konzentration in den Nagel penetrieren (WO 96/19186), wurde nun überraschend gefunden, dass Kombinationen von topischen mit systemischen Antimykotika in einer Lackgrundlage nach topischer Applikation ohne die oben genannte Maßnahme und den genannten Zusatz in der Lage sind, in therapeutisch wirksamen Konzentrationen in und durch den Nagel zu penetrieren. Ferner weisen Patienten, die mit einem systemischen Antimykotikum behandelt wurden nur vergleichsweise geringe Konzentrationen des Antimykotikums im Fuß- oder Fingernagel auf.

Die Erfindung betrifft daher eine pharmazeutische Zubereitung, enthaltend einen wasserunlöslichen Filmbildner, mindestens ein systemisches Antimykotikum aus der Gruppe Itraconazol, Terbinafin und Fluconazol und/oder physiologisch verträgliche Salze von Itraconazol, Terbinafin und Fluconazol, und mindestens ein topisches Antimykotikum aus der Gruppe Ciclopirox, 6-(2,4,4-trimethylpentyl)-1-hydroxy-4-methyl-2 (1H)-pyridon, Amorolfin und Butenafin und/oder physiologisch verträgliche Salze von Ciclopirox, 6-(2,4,4-trimethylpentyl)-1-hydroxy-4-methyl-2(1 H)-pyridon, Amorolfin und Butenafin, oder eine Mischung aus mehreren der obengenannten Antimykotika oder deren Salze.

Bevorzugt sind pharmazeutische Zubereitungen, die Ciclopirox (das auch als 6-Cyclohexyl-1-hydroxy-4-methyl-2 (1 H)-pyridon bezeichnet wird) und Itraconazol, oder Butenafin Hydrochlorid und Fluconazol, oder Ciclopirox und Fluconazol, oder Amorolfin Hydrochlorid und Terbinafin Hydrochlorid, als Antimykotikum enthalten.

Die obengenannten topischen oder systemischen Antimykotika werden sowohl in freier Form als auch als physiologisch verträgliche Salze eingesetzt. Kommen bei den Salzen organische Basen zur Anwendung, so werden vorzugsweise schwer flüchtige Basen eingesetzt, beispielsweise niedrigmolekulare Alkanolamine wie Ethanolamin, Diethanolamin, N-Ethylethanolamin, N-Methyl-diethanolamin, Triäthanolamin, Diethylamino-ethanol, 2-Amino-2-methyl-n-propanol, Dimethylaminopropanol, 2-Amino-2-methyl-propandiol, Tri-isopropanolamin. Als weitere schwerer flüchtige Basen seien beispielsweise erwähnt Ethylendiamin, Hexametylendiamin, Morpholin, Piperidin, Piperazin, Cyclohexylamin, Tributylamin, Dodecylamin, N,N-Dimethyl-dodecylamin, Stearylamin, Oleylamin, Benzylamin, Dibenzylamin, N-Ethylbenzylamin, Dimethylstearylamin, N-Methylmorpholin, N-Methylpiperazin, 4-Methylcyclohexylamin, N-Hydroxyethyl-morpholin. Auch die Salze quartärer Ammoniumhydroxide wie Trimethylbenzyl-ammonium-hydroxid, Tetramethylammoniumhydroxid oder Tetraethylammoniumhydroxid können verwendet werden, ferner Guanidin und seine Abkömmlinge, insbesondere seine Alkylierungsprodukte. Es ist jedoch auch möglich, als Salzbildner beispielsweise niedrigmolekulare Alkylamine wie Methylamin, Ethylamin oder Triethylamin einzusetzen. Auch Salze mit anorganischen Kationen, beispielsweise Alkalisalze, insbesondere Natrium-, Kalium- oder Ammonium-Salze, insbesondere Hydrochloride, Erdalkalisalze wie insbesondere das Magnesium- oder Calciumsalz, sowie Salze mit zwei- bis vierwertigen Kationen, beispielsweise das Zink-, Aluminiumoder Zirkonium-Salz kommen für die erfindungsgemäß einzusetzenden Verbindungen in Betracht.

Die Erfindung betrifft auch die Verwendung von einem wasserunlöslichen Filmbildner, mindestens einem systemischen Antimykotikums aus der Gruppe Itraconazol, Terbinafin und Fluconazol und/oder physiologisch verträgliche Salze von Itraconazol, Terbinafin und Fluconazol, und mindestens einem topischen Antimykotikums aus der Gruppe Ciclopirox, 6-(2,4,4-trimethylpentyl)-1-hydroxy-4-methyl-2 (1H)-pyridon, Amorolfin und Butenafin und/oder physiologisch verträgliche Salze von Ciclopirox, 6-(2,4,4-trimethylpentyl)-1-hydroxy-4-methyl-2(1H)-pyridon Amorolfin und Butenafin, oder eine Mischung aus mehreren der obengenannten Antimykotika oder deren Salze zur Herstellung einer topischen pharmazeutischen Zubereitung zur prophylaktischen und therapeutischen Behandlung von Pilzerkrankungen der Fuß- und Fingernägel.

Der Gehalt an topischem und systemischem Wirkstoff in den erfindungsgemäßen Lackzubereitungen ist von der Struktur eines jeden Wirkstoffes und damit von dessen Freigabe aus dem Lackfilm, seinem Penetrationsverhalten im Nagel und seinen antifungischen Eigenschaften abhängig.

In den erfindungsgemäßen pharmazeutischen Zubereitungen für die Therapie der Onychomykosen, die als Nagellack eingesetzt werden, also die Lösemittel enthaltende Anwendungsform, ist der topische Wirkstoff im allgemeinen in einer Menge von 0,25 bis 20 Gewichtsprozent, vorzugsweise 2 bis 15 Gewichtsprozent enthalten. Der Gehalt an "systemischem" Wirkstoff beträgt im allgemeinen 0,05 bis 10 Gewichtsprozent, vorzugsweise 0,1 bis 5 Gewichtsprozent.

Die erfindungsgemäßen Nagellacke enthalten außer dem in einem Lösemittel oder Lösemittelgemisch gelösten Wirkstoff als notwendige Bestandteile noch einen oder mehrere Filmbildner, die nach dem Trocknen der Zubereitung einen wasserunlöslichen Film auf dem Nagel bilden.

Als Filmbildner eignen sich z.B. Stoffe auf der Basis von Cellulosenitrat oder physiologisch unbedenkliche Polymerisate, wie sie z.B. in Kosmetika üblich sind, vorzugsweise als Gemisch mit Cellulosenitrat. Genannt seien z.B. Polyvinylacetat und partiell verseiftes Polyvinylacetat, Mischpolymerisate aus Vinylacetat einerseits und Acrylsäure oder Crotonsäure oder Maleinsäuremonoalkylester andererseits, temäre Mischpolymerisate aus Vinylacetat einerseits und Crotonsäure und Vinylneodecanoat, oder Crotonsäure und Vinylpropionat andererseits, Mischpolymerisate aus Methylvinyläther und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und Methacrylsäurealkylester, Mischpolymerisate aus Acrylsäure und Methacrylsäure oder Acrylsäurealkylester oder Methacrylsäurealkylester, Polyvinylacetate und Polyvinylbutyrale, alkylsubstituierte Poly-N-vinylpyrrolidone, Alkylester aus Mischpolymerisaten aus Oelfinen und Maleinsäureanhydrid und Umsetzungsprodukte von Kolophonium mit Acrylsäure. In den Estern sind die Alkylreste gewöhnlich kurzkettig und haben meistens nicht mehr als vier C-Atome.
Bevorzugt werden Zubereitung eingesetzt, enthaltend einen wasserunlöslichen Filmbildner aus der Gruppe Copolymerisat aus Ethylacrylat-Methylmethacrylat-Trimethylammonioethylmethacrylat-chlorid, Mischpolymerisat aus Acryl- und Methacrylsäureester mit Anteilen von Trimethylammoniumethylmethacrylatchlorid, Mischpolymerisat aus Methylvinylether und Maleinsäuremonobutylester, Polymerisat aus Polyvinylbutyral und Cellulosenitrat oder Copolymer aus Methacrylsäure und Ethylacrylat.

Als physiologisch unbedenkliche Lösemittel kommen Stoffe wie in Kosmetika übliche Kohlenwasserstoffe, halogenierte Kohlenwasserstoffe, Alkohole, Ether, Ketone und Ester in Betracht, insbesondere Essigsäureester von einwertigen Alkoholen wie Ethyl- und Butylacetat, gegebenenfalls im Gemisch mit aromatischen Kohlenwasserstoffen wie Toluol und/oder Alkoholen wie Ethanol oder Isopropanol.

Die Kombination der Lösemittel ist bekanntlich von entscheidender Bedeutung für die Trockenzeit, Verstreichbarkeit und andere wichtige Eigenschaften des Lackes bzw. des Lackfilms. Das Lösemittelsystem besteht vorzugsweise aus einer optimalen Mischung aus Niedrigsiedern (= Lösemittel mit einem Siedepunkt bis 100°C) und Mittelsiedern (= Lösemittel mit einem Siedepunkt bis 150°C), gegebenenfalls mit einem kleinen Anteil Hochsiedern (= Lösemittel mit einem Siedepunkt bis 200°C).

Die erfindungsgemäßen Nagellacke können weiterhin in Kosmetika gebräuchliche Zusätze enthalten, wie Weichmacher auf Phthalat- oder Campherbasis, Farbstoffe bzw. Farbpigmente, Perlglanzmittel, Sedimentationsverzögerer, Sulfonamidharze, Silikate, Riechstoffe, Netzmittel wie Natriumdioctylsulfosuccinat, Lanolinderivate, Lichtschutzmittel, wie 2-Hydroxy-4-methoxybenzophenon, antibakteriell wirksame Substanzen und Stoffe mit keratolytischer und/oder keratoplastischer Wirkung, wie Harnstoff, Allantoin, Enzyme und Salizylsäure.

Gefärbte oder pigmentierte Nagellacke haben z.B. den Vorteil, dass die erfindungsgemäße Zubereitung dem Schönheitsempfinden des Patienten angepasst werden kann und die bestehenden Nagelveränderungen für Dritte nicht unmittelbar sichtbar sind.

Ein Verfahren zur Herstellung der wasserunlöslichen Nagellacke besteht darin, dass man die physiologisch unbedenklichen Lackgrundlage in gelöster Form mit den Antimykotika mischt und die Zubereitung soweit erforderlich weiter verarbeitet.

Jeweils bezogen auf die Menge der nicht flüchtigen Bestandteile, das ist die Summe der Filmbildner, der gegebenenfalls vorhandene Pigmente, Weichmacher und anderen nicht flüchtigen Zusätze sowie der eingesetzten wirksamen Antimykotika, sind in den erfindungsgemäßen pharmazeutischen Zubereitungen die Antimykotika im allgemeinen in einer Menge von 2 bis 80 Gewichtsprozent, vorzugsweise von 10 bis 60 Gewichtsprozent und insbesondere von 20 bis 40 Gewichtsprozent enthalten.

Mit den erfindungsgemäßen Lackzubereitungen läßt sich bei der Behandlung von Onychomykosen eine durchgreifende Heilung - ohne das Auftreten von systemischen Nebenwirkungen und Arzneimittelinteraktionen - erzielen. Im Hinblick auf die bisherigen Therapieerfahrungen ist dies ein überaus wichtiger Befund. Ein weiterer Vorteil ist die wesentlich kürzere Behandlungszeit mit der erfindungsgemäßen pharmazeutischen Zubereitung. Dies zeigt sich insbesondere in den wesentlich höheren Konzentrationen der systemischen Antimykotika im Nagel nach topischer Applikation.

Die erfindungsgemäßen pharmazeutischen Zubereitungen eignen sich auch zur prophylaktischen Anwendung gegen Onychomykosen, wobei ein ausreichend hohes Wirkstoffdepot im Nagel erreicht wird, so dass es im Falle einer Pilzkontamination nicht zum Ausbruch einer durch Pilze hervorgerufenen Nagelerkrankung kommt. Die zur Prophylaxe verwendeten pharmazeutischen Zubereitungen enthalten geringere Mengen von den therapeutisch eingesetzten Antimykotika. Bevorzugt wird in den pharmazeutischen Zubereitungen für die Prophylaxe der Onychomykosen, die als Nagellack eingesetzt werden, also die Lösemittel enthaltende Anwendungsform, der Wirkstoff im allgemeinen in einer Menge von 0,25 bis 4 Gewichtsprozent, vorzugsweise 1 bis 4 Gewichtsprozent eingesetzt. Der Gehalt an "systemischem" Wirkstoff beträgt im allgemeinen 0,05 bis 3 Gewichtsprozent, vorzugsweise 0,1 bis 1 Gewichtsprozent. Im Vergleich zur systemischen Monotherapie ist zum Aufbau eines entsprechenden Wirkstoffdepots eine wesentlich geringere Substanzmenge erforderlich.

Die Erfindung betrifft ferner den Einsatz der erfindungsgemäßen Zubereitungen in der Kosmetik.

Die vorliegende Erfindung wird durch die folgenden Beispiele näher erläutert, jedoch nicht auf diese beschränkt. Soweit nichts anderes vermerkt, sind die Mengenangaben auf das Gewicht bezogen.

### Beispiel 1

| | |
|---|---|
| Amorolfinhydrochlorid | 5,0% |
| Terbinafrnhydrochlorid | 2,5% |
| Mischpolymerisat aus Acryl- und Methacrylsäureester mit Anteilen von Trimethylammoniumethylmethacrylatchlorid (z.B. EUDRAGIT RL 100) | 20,0% |
| Isopropylmyristat | 2,5% |
| Isopropylalkohol | 70,0% |

### Beispiel 2

| | |
|---|---|
| Butenafinhydrochlorid | 5,0% |
| Fluconazol | 5,0% |
| Mischpolymerisat aus Methylvinylether und Maleinsäuremonobutylester | 25,0% |
| Ethanol 96% | 65,0% |

### Beispiel 3

| | |
|---|---|
| Ciclopirox | 8,0% |
| Itraconazol | 0,5% |
| Isopropylmyristat | 5,0% |
| 1,2-Propylenglykol | 4,0% |
| Mischpolymerisat aus Methylvinylether und Maleinsäuremonobutylester | 7,5% |
| Ethylacetat | 25,0% |
| Isopropylalkohol | 50,0% |

### Beispiel 4

| | |
|---|---|
| Ciclopirox | 7,5% |
| Fluconazol | 5,0% |
| Mischpolymerisat aus Methylvinylether und Maleinsäuremonobutylester | 15,0% |
| Ethylacetat | 30% |
| Ethanol 96% | 42,5% |

### Beispiel 5

Die Wirkstoffkombination nach Applikation der erfindungsgemäßen Zubereitungen im Nagel wurde im beim Schneiden der Nägel von Probanden anfallenden distalen Nagelmaterial nach Extraktion durch HPLC Messungen bestimmt.

Nach 6 Wochen 2 x wöchentlicher Behandlung wurden dabei für die Zubereitung gemäß Beispiel 3 folgende Werte ermittelt:

| | |
|---|---|
| Itraconazol | 800 µg/g Nagel |
| Ciclopirox | 140 µg/g Nagel |

Nach oraler Gabe von täglich 100 mg Itraconazol für 3 Monate wird im Stand der Technik eine Itraconazol Konzentration in der distalen Nagelplatte von 100 µg/g Nagel ausgewiesen.
Daher lassen sich durch die erfindungsgemäße pharmazeutische Zubereitung in kurzer Zeit etwa acht mal höhere Konzentrationen von Itraconazol im Nagel erreichen als durch systemische Behandlung.

### Beispiel 6

Nach 2 Wochen 2 x wöchentlicher Behandlung wurden für die Zubereitung gemäß Beispiel 4 folgende Werte ermittelt:

| | |
|---|---|
| Fluconazol | 17 µg/g Nagel |
| Ciclopirox | 92 µg/g Nagel |

## Patentansprüche

1. Zubereitung, enthaltend einen wasserunlöslichen Filmbildner, und eine Wirkstoffkombination aus mindestens einem Antimykotikum aus der Gruppe Itraconazol, Terbinafin und Fluconazol und/oder physiologisch verträgliche Salze von Itraconazol, Terbinafin und Fluconazol, und
mindestens ein Antimykotikum aus der Gruppe Ciclopirox, 6-(2,4,4-trimethylpentyl)-1-hydroxy-4-methyl-2(1H)-pyridon, Amorolfin und Butenafin und/oder physiologisch verträgliche Salze von Ciclopirox, 6-(2,4,4-trimethylpentyl)-1-hydroxy-4-methyl-2(1 H)-pyridon, Amorolfin und Butenafin, oder eine Mischung aus mehreren der obengenannten Antimykotika oder deren Salze.

2. Zubereitung gemäß Anspruch 1, enthaltend als Antimykotika Ciclopirox und Itraconazol, oder
Butenafin Hydrochlorid und Fluconazol, oder
Ciclopirox und Fluconazol, oder
Amorolfin Hydrochlorid und Terbinafin Hydrochlorid.

3. Zubereitung gemäß Anspruch 1 oder 2, enthaltend als physiologisch unbedenkliche Lackgrundlage einen Filmbildner auf der Basis von Cellulosenitrat, Polyvinylacetat und partiell verseiftes Polyvinylacetat, Mischpolymerisate aus Vinylacetat einerseits und Acrylsäure oder Crotonsäure oder
Maleinsäuremonoalkylester andererseits, ternäre Mischpolymerisate aus Vinylacetat einerseits und Crotonsäure und Vinylneodecanoat, oder Crotonsäure und Vinylpropionat andererseits, Mischpolymerisate aus Methylvinyläther und Maleinsäuremonoalkylester, insbesondere als Maleinsäuremonobutylester, Mischpolymerisate aus Fettsäurevinylester und Acrylsäure oder Methacrylsäure, Mischpolymerisate aus N-Vinylpyrrolidon, Methacrylsäure und
Methacrylsäurealkylester, Mischpolymerisate aus Acrylsäure und Methacrylsäure oder Acrylsäurealkylester oder Methacrylsäurealkylester, Polyvinylacetate und Polyvinylbutyrale, alkylsubstituierte Poly-N-vinylpyrrolidone, Alkylester aus Mischpolymerisaten aus Oelfinen und Maleinsäureanhydrid und
Umsetzungsprodukte von Kolophonium mit Acrylsäure, worin in den Estern die Alkylreste gewöhnlich kurzkettig sind und nicht mehr als vier Kohlenstoffatome aufweisen.

4. Zubereitung gemäß Anspruch 3, enthaltend als wasserunlöslichen Filmbildner ein Copolymerisat aus Ethylacrylat-Methytmethacrylat-Trimethylammonioethylmethacrylat-chlorid, Mischpolymerisat aus Acryl- und Methacrylsäureester mit Anteilen von Trimethylammoniumethy(methacrylatchlorid, Mischpolymerisat aus Methylvinylether und Maleinsäuremonobutylester, Polymerisat aus Polyvinylbutyral und Cellulosenitrat oder ein Copolymer aus Methacrylsäure und Ethylacrylat.

5. Zubereitung gemäß einem oder mehreren der Ansprüche 1 bis 4, enthaltend ein Antimykotikum aus der Gruppe Ciclopirox, 6-(2,4,4-timethylpentyl)-1-hydroxy-4-methyl-2 (1H)-pyridon, Amorolfin und Butenafin in einer Menge von 0,25 bis 20 Gewichtsprozent, vorzugsweise 2 bis 15 Gewichtsprozent, und ein Antimykotikum aus der Gruppe Itraconazol, Terbinafin und Fluconazol in einer Menge von 0,05 bis 10 Gewichtsprozent, vorzugsweise 0,1 bis 5 Gewichtsprozent.

6. Verfahren zur Herstellung der pharmazeutischen Zubereitung gemäß einem oder mehreren der Ansprüche 1 bis 5, **dadurch gekennzeichnet, dass** man die physiologisch unbedenklichen Lackgrundlage in gelöster Form mit den Antimykotika mischt und die Zubereitung soweit erforderlich weiter verarbeitet.

7. Verwendung der Zubereitung gemäß einem oder mehreren der Ansprüche 1 bis 5 zur Herstellung einer pharmazeutischen zur prophylaktischen und therapeutischen Behandlung von Pilzerkrankungen der Fuß- und Fingernägel.

8. Verwendung von einem wasserunlöslichen Filmbildner gemäß der Ansprüche 1 bis 5, mindestens einem Antimykotikums aus der Gruppe Itraconazol, Terbinafin und Fluconazol und/oder physiologisch verträgliche Salze von Itraconazol, Terbinafin und Fluconazol, und mindestens einem Antimykotikums aus der Gruppe Ciclopirox, 6-(2,4,4-trimethylpentyl)-1-hydroxy-4-methyl-2 (1H)-pyridon, Amorolfin und Butenafin und/oder physiologisch verträgliche Salze von Ciclopirox, 6-(2,4,4-trimethylpentyl)-1-hydroxy-4-methyl-2 (1 H)-pyridon Amorolfin und Butenafin, oder eine Mischung aus mehreren der obengenannten Antimykotika oder deren Salze zur Herstellung einer topischen pharmazeutischen Zubereitung zur prophylaktischen und therapeutischen Behandlung von Pilzerkrankungen der Fuß- und Fingernägel.

9. Verwendung einer Zubereitung gemäß einem oder mehreren der Ansprüche 1 bis 5 in der Kosmetik.

## Claims

1. A preparation comprising a water-insoluble film former, and a combination of active ingredients comprising at least one antimycotic from the group of itraconazole, terbinafine and fluconazole and/or physiologically tolerated salts of itraconazole, terbinafine and fluconazole, and
at least one antimycotic from the group of ciclopirox, 6-(2,4,4-trimethylpentyl)-1-hydroxy-4-methyl-2(1H)-pyridone,
amorolfine and butenafine and/or physiologically tolerated salts of ciclopirox, 6-(2,4,4-trimethylpentyl)-1-hydroxy-4-methyl-2(1H)-pyridone, amorolfine and butenafine, or a mixture of more than one of the abovementioned antimycotics or salts thereof.

2. The preparation as claimed in claim 1, comprising as antimycotics ciclopirox and itraconazole, or
butenafine hydrochloride and fluconazole, or
ciclopirox and fluconazole, or
amorolfine hydrochloride and terbinafine hydrochloride.

3. The preparation as claimed in claim 1 or 2, comprising as physiologically acceptable lacquer base a film former based on cellulose nitrate, polyvinyl acetate and partially hydrolyzed polyvinyl acetate, copolymers of vinyl acetate on the one hand and acrylic acid or crotonic acid or monoalkyl maleate on the other hand, ternary copolymers of vinyl acetate on the one hand and crotonic acid and vinyl neodecanoate, or crotonic acid and vinyl propionate on the other hand, copolymers of methyl vinyl ether and monoalkyl maleate, in particular as monobutyl maleate, copolymers of fatty acid vinyl ester and acrylic acid or methacrylic acid, copolymers of N-vinylpyrrolidone, methacrylic acid and alkyl methacrylate, copolymers of acrylic acid and methacrylic acid or alkyl acrylate or alkyl methacrylate, polyvinyl acetates and polyvinyl butyrals, alkyl-substituted poly-N-vinylpyrrolidones, alkyl esters from copolymers of olefins and maleic anhydride and products of the reaction of rosin with acrylic acid; the alkyl radicals in the esters are usually short-chain and have not more than four carbon atoms.

4. The preparation as claimed in claim 3, comprising as water-insoluble film former a copolymer of ethyl acrylate/methyl methacrylate/trimethylammonioethyl methacrylate chloride, copolymer of acrylic and methacrylic ester with proportions of trimethylammoniumethyl methacrylate chloride, copolymer of methyl vinyl ether and monobutyl maleate, polymer of polyvinylbutyral and cellulose nitrate or a copolymer of methacrylic acid and ethyl acrylate.

5. The preparation as claimed in one or more of claims 1 to 4, comprising an antimycotic from the group of ciclopirox, 6-(2,4,4-trimethylpentyl)-1-hydroxy-4-methyl-2(1H)-pyridone, amorolfine and butenafine in an amount of from 0.25 to 20 percent by weight, preferably 2 to 15 percent by weight, and an antimycotic from the group of itraconazole, terbinafine and fluconazole in an amount of from 0.05 to 10 percent by weight, preferably 0.1 to 5 percent by weight.

6. A process for producing the pharmaceutical preparation as claimed in one or more of claims 1 to 5, which comprises mixing the physiologically acceptable lacquer base in dissolved form with the antimycotics, and further processing the preparation where necessary.

7. The use of the preparation as claimed in one or more of claims 1 to 5 for producing a pharmaceutical preparation for the prophylactic and therapeutic treatment of fungal infections of the toenails and fingernails.

8. The use of a water-insoluble film former as set forth in claims 1 to 5, at least one antimycotic from the group of itraconazole, terbinafine and fluconazole and/or physiologically tolerated salts of itraconazole, terbinafine and fluconazole, and at least one antimycotic from the group of ciclopirox, 6-(2,4,4-trimethylpentyl)-1-hydroxy-4-methyl-2(1H)-pyridone, amorolfine and butenafine and/or physiologically tolerated salts of ciclopirox, 6-(2,4,4-trimethylpentyl)-1-hydroxy-4-methyl-2(1H)-pyridone, amorolfine and butenafine, or a mixture of more than one of the abovementioned antimycotics or salts thereof, for producing a topical pharmaceutical preparation for the prophylactic and therapeutic treatment of fungal infections of the toenails and fingernails.

9. The use of a preparation as claimed in one or more of claims 1 to 5 in cosmetics.

## Revendications

1. Préparation renfermant un agent filmogène insoluble et une combinaison de substances actives d'au moins un antifongique pris dans le groupe comprenant l'itraconazol, la terbinafine et le fluconazol et/ou des sels physiologiquement tolérés d'itraconazol, de terbinafine et de fluconazol, et au moins un antifongique pris dans le groupe comprenant le ciclopirox, la 6-(2,4,4-triméthylpentyl)-1-hydroxy-4-méthyl-2(1H)-pyridone, l'amorolfine et la buténafine, ou un mélange de plusieurs antifongiques cités ci-dessus ou leurs sels.

2. Préparation selon la revendication 1, renfermant en tant qu'antifongique
le ciclopirox et l'itraconazol, ou
la buténafine et le fluconazol, ou
le ciclopirox et le fluconazol ou
le chlorhydrate d'amorolfine et le chlorhydrate de terbinafine.

3. Préparation selon la revendication 1 ou 2, renfermant en tant que substrat de vernis physiologiquement inoffensif un agent filmogène à base de nitrate de cellulose, de poly(acétate de vinyle) et de poly(acétate de vinyle) partiellement saponifié, des copolymères d'acétate de vinyle d'une part et d'acide acrylique ou d'acide crotonique ou de maléate de monoalkyle d'autre part, des copolymères ternaires d'acétate de vinyle d'une part et d'acide crotonique et néodécanoate de vinyle, ou d'acide crotonique et de propionate de vinyle d'autre part, des copolymères d'éther méthylvinylique et de maléate de monoalkyle, en particulier en tant que maléate de monobutyle, des copolymères d'esters vinyliques d'acides gras et d'acide acrylique ou d'acide méthacrylique, des copolymères de N-vinylpyrrolidone, d'acide méthacrylique et de méthacrylate d'alkyle, des copolymères d'acide acrylique et d'acide méthacrylique ou d'acrylate d'alkyle ou de méthacrylate d'alkyle, des poly(acétates de vinyle) et des poly(butyrals de vinyle), des poly-N-vinylpyrrolidones substituées par un substituant alkyle, des esters d'alkyles de copolymères d'oléfines et d'anhydride maléique et des produits de réaction de la cellophane et de l'acide acrylique, les restes alkyles dans les esters présentent usuellement une chaîne courte et ne présentent pas plus de quatre atomes de carbone.

4. Préparation selon la revendication 3, renfermant des agents filmogènes insolubles dans l'eau, un copolymère d'acrylate d'éthyle-méthacrylate de méthyle-chlorure de triméthylammonioéthylméthacrylate, un copolymère d'acrylate et de méthacrylate renfermant des taux de chlorure de triméthylammoniuméthylméthacrylate, un copolymère d'éther méthylvinylique et de maléate de monobutyle, un polymère de poly(butyral de vinyle) et de nitrate de cellulose ou un copolymère d'acide méthacrylique et d'acrylate d'éthyle.

5. Préparation selon une ou plusieurs des revendications 1 à 4, renfermant un agent antifongique pris dans le groupe comprenant le ciclopirox, la 6-(2,4,4-triméthylpentyl)-1-hydroxy-4-méthyl-2(1H)-pyridone, l'amorolfine et la buténafine dans une quantité de 0,25 à 20 % en masse, de préférence de 2 à 15 % en masse et un agent antifongique pris dans le groupe comprenant l'itraconazol, la terbinafine et le fluconazol dans une quantité de 0,05 à 10 % en masse, de préférence de 0,1 à 5 % en masse.

6. Procédé pour la préparation de la préparation pharmaceutique selon une ou plusieurs des revendications 1 à 5, **caractérisé en ce qu'**on mélange la base de vernis physiologiquement inoffensive sous forme dissoute avec l'agent antifongique et, si nécessaire, on soumet la préparation à un traitement complémentaire.

7. Utilisation de la préparation selon une ou plusieurs des revendications 1 à 5 pour la préparation d'un traitement pharmaceutique préventif et thérapeutique de mycoses d'ongles d'orteils et de doigts.

8. Utilisation d'un agent filmogène insolubles dans l'eau selon les revendications 1 à 5, d'au moins un agent antifongique pris dans le groupe comprenant l'itraconazol, la terbinafine et le fluconazol et/ou des sels physiologiquement tolérés d'itraconazol, de terbinafine et de fluconazol, et au moins un antifongique pris dans le groupe comprenant le ciclopirox, la 6-(2,4,4-triméthylpentyl)-1-hydroxy-4-méthyl-2(1H)-pyridone, l'amorolfine et la buténafine, et/ou des sels physiologiquement tolérés de ciclopirox, de 6-(2,4,4-triméthylpentyl)-1-hydroxy-4-méthyl-2(1H)-pyridone, d'amorolfine et de buténafine, ou un mélange de plusieurs antifongiques cités ci-dessus ou leurs sels, pour la préparation d'une préparation pharmaceutique topique pour le traitement préventif et thérapeutique de mycoses d'ongles d'orteils et de doigts.

9. Utilisation d'une préparation selon une ou plusieurs des revendications 1 à 5 en cosmétique.
